Europäisches Patentamt
European Patent Office
Office européen des brevets

(11) Veröffentlichungsnummer: **0 163 984**
**A2**

# EUROPÄISCHE PATENTANMELDUNG

(21) Anmeldenummer: **85105799.2**

(22) Anmeldetag: **11.05.85**

(51) Int. Cl.⁴: **A 61 K 31/44**, A 61 K 31/535, A 61 K 45/06 // (A61K31/535, 31:44),(A61K31/44, 31:165),(A61K31/44, 31:135),(A61K31/44, 31:40)

(30) Priorität: **23.05.84 DE 3419131**

(43) Veröffentlichungstag der Anmeldung: **11.12.85 Patentblatt 85/50**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Posanski, Ulrich, Dr., Ahornweg 65, D-5064 Rösrath (DE)**

(54) **Dihydropyridinkombinationspräparate und Verfahren zu ihrer Herstellung.**

(57) Die Erfindung betrifft neue Dihydropyridinkombinationspräparate in fester Form mit verbesserter Bioverfügbarkeit, enthaltend schwer lösliche Dihydropyridine und β-Blocker, Verfahren zu ihrer Herstellung und ihre Verwendung als Arzneimittel, insbesondere als Coronar- und Blutdruckmittel.

BAYER AKTIENGESELLSCHAFT
Konzernverwaltung RP
Patentabteilung

5090 Leverkusen, Bayerwerk
V (Pha)
KS/by/c

Dihydropyridinkombinationspräparate und Verfahren zu ihrer Herstellung

Die Erfindung betrifft neue Dihydropyridinkombinationspräparate in fester Form mit verbesserter Bioverfügbarkeit, enthaltend schwer lösliche Dihydropyridine und ß-Blocker, Verfahren zu ihrer Herstellung und ihre Verwendung als Arzneimittel, insbesondere als Coronar- und Blutdruckmittel.

Es ist bereits bekannt geworden, daß Dihydropyridine sehr starke kreislaufbeeinflussende Wirkungen besitzen (vgl. britisches Patent 1 358 951). Aufgrund der schweren Löslichkeit vieler Dihydropyridine treten bei der galenischen Zubereitung von Arzneispezialitäten eine Reihe von Schwierigkeiten auf, wie aus zahlreichen Publikationen und Patentanmeldungen über spezielle Formulierungen dieser Wirkstoffe ersichtlich wird. In dem britischen Patent 1 456 618 werden z.B. feste Arzneizubereitungen beschrieben und beansprucht, welche ebenfalls eine gute Bioverfügbarkeit von schwer löslichen Dihydropyridinen gewährleisten sollen. Auch in der DT-OS 2 822 882

werden feste Arzneiformen beschrieben, bei denen durch den Einsatz bestimmter Lösungsvermittler und oberflächenaktiver Substanzen die Schwerlöslichkeit der Wirkstoffe kompensiert werden soll. Auch in der europäischen Offenlegungsschrift 1 247 soll die Resorbierbarkeit von Dihydropyridinen durch die Verwendung von Polyethylenglykol und bestimmter poröser Trägersubstanzen verbessert werden.

In der Literatur werden ebenfalls verschiedene Methoden zur Verbesserung der Bioverfügbarkeit, die unmittelbar mit einer verbesserten Löslichkeit verbunden ist, beschrieben. Neben der Änderung der chemischen Struktur, z.B. durch Einbau von löslichkeitsverbessernden Substituentengruppen sind zahlreiche Methoden beschrieben, die eine Änderung der physikalischen Eigenschaften der Wirkstoffe betreffen. So wird z.B. die Zerkleinerung von Wirkstoffpartikeln, Veränderung der Kristallstruktur, die Herstellung von Salzformen, Zugabe von Netzmitteln, Komplexbildung mit anderen Substanzen, Verwendung von biologischen Carriern, Erstellung fester Dispersionen (Copräzipitate) oder die Herstellung von Oberflächenadsorbaten vorgeschlagen (vgl. S.H. Yalkowskay, Drugs and the pharmaceutical science, 12, 135-180 (1981) und J. Polderman, Formulation and preparation of dosage forms, Elsevier/North-Holland Biomedical Press, 1977, 215-219).

Alle bisherigen Versuche, die schlechte Löslichkeit von Dihydropyridinen durch bestimmte Maßnahmen zu kompensieren und gleichzeitig eine gute Bioverfügbarkeit zu gewährleisten, besitzen eine Reihe von Nachteilen. Der Einsatz von oberflächenaktiven Substanzen, Lösungsvermittlern und bestimmten Trägerstoffen, die eine besondere Oberfläche haben, z.B. porös sind, führt häufig zu Verabreichungsformen, bei denen die Präparate unerwünscht groß sind. Zur Erleichterung des Schluckens werden solche Tabletten oder Kapseln häufig in spezifische Formen wie z.B. Ellipsoide oder Längsformen überführt, was jedoch bei Präparaten mit einem Gewicht über 400 mg auch nicht immer zu befriedigenden Ergebnissen führt. Auch das häufigere Einnehmen von kleineren Präparaten stellt keine befriedigende Lösung dar.

Für Arzneizubereitungen gilt, daß sowohl die Zahl als auch die Menge der Hilfs- und Trägerstoffe möglichst niedrig gehalten werden soll. Beim Vergleich zweier Arzneispezialitäten wird man immer demjenigen Präparat den Vorzug geben, welches neben dem Wirkstoff möglichst wenig Hilfsstoffe und Zuschlagstoffe enthält, um unerwünschte biologische Nebenwirkungen weitgehend zu vermeiden.

Die vorliegende Erfindung betrifft neue feste Kombinationspräparate mit verbesserter Bioverfügbarkeit, enthaltend 1 Gewichtsteil eines schwer löslichen Dihydropyridins der allgemeinen Formel (I)

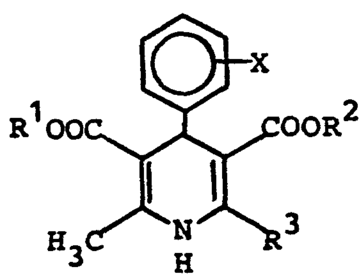

(I)

in der

$R^1$ $C_1$-$C_4$-Alkyl, gegebenenfalls substituiert durch $C_2$-$C_3$-Alkoxy,

$R^2$ $C_1$-$C_{10}$-Alkyl, gegebenenfalls substituiert durch $C_1$-$C_3$-Alkoxy, Trifluormethyl, N-Methyl-N-benzylamino,

$R^3$ $C_1$-$C_4$-Alkyl, Cyano, Hydroxymethyl und

X 2- bzw. 3-Nitro, 2,3-Dichlor, 2,3 Ringglied bestehend aus =N-O-N=,

bedeuten,

und 0,5 bis 10 Gewichtsteile eines ß-Blockers und gegebenenfalls weitere übliche Hilfs- und Trägerstoffe.

Von besonderem Interesse sind erfindungsgemäße Kombinationspräparate, die einen ß-Blocker aus der Gruppe Acebutolol, Atenolol, Nadolol, Propranolol, Pindolol oder Timolol enthalten.

Insbesondere seien Acebutolol und Atenolol genannt.

Von besonderem Interesse sind erfindungsgemäße Kombinationspräparate enthaltend 1 Gewichtsteil eines Dihydropyridins der allgemeinen Formel I, in welcher $R^1$ und $R^2$ immer voneinander verschieden sind, insbesondere Nisoldipin, Nimodipin oder Nitrendipin, und 1 bis 5 Gewichtsteile eines ß-Blockers. Als bevorzugte ß-Blocker seien Acebutolol und Atenolol genannt.

Die Herstellung der erfindungsgemäßen Kombinationspräparate erfolgt, indem man den Dihydropyridinwirkstoff und

**den ß-Blockerwirkstofff mit bekannten Hilfsstoffen gemeinsam granuliert mittels eines Feuchtgranulationsverfahrens oder eines Trockenkompaktierverfahrens und das erhaltene Granulat gegebenenfalls mit mindestens einem Tablettenhilfstoff zu Tabletten verpreßt.**

Die Herstellung der erfindungsgemäß verwendbaren Dihydropyridine der allgemeinen Formel I erfolgt vorzugsweise durch Umsetzung von Ylidenverbindungen der allgemeinen Formel II

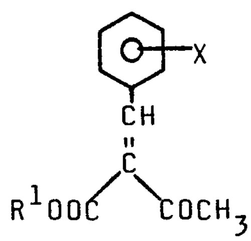

(II)

mit Enaminverbindungen der allgemeinen Formel III

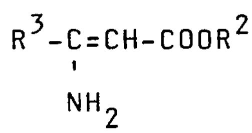

(III)

wobei $R^1$, $R^2$, $R^3$ und X die für Formel I angegebene Bedeutung haben,

in Gegenwart von niederen aliphatischen Alkoholen als Lösungsmittel bei Temperaturen von 40 - 100°C.

Eine vorteilhafte Variante dieser Umsetzung liegt in der Verwendung eines geringen molaren Überschusses der Enaminverbindung der allgemeinen Formel III und einer Entfernung des sich bildenden Reaktionswassers, gegebenenfalls durch azeotrope Destillation, während der Umsetzung.

Die Ylidenverbindung der Formel II wird vorzugsweise hergestellt durch Reaktion molarer Mengen eines Ketocarbonsäureesters mit einem aromatischen Aldehyd in niederen Alkoholen als Lösungsmittel, insbesondere Alkohol mit 1 bis 4 Kohlenstoffen, in Gegenwart von katalytischen Mengen Piperidinacetat bei Temperaturen zwischen 20 und 70°C.

Die Herstellung der Enaminverbindung der allgemeinen Formel III erfolgt vorzugsweise durch Umsetzung von Ketocarbonsäureestern und Ammoniak in niederen Alkoholen, vorzugsweise Isopropanol, bei Temperaturen zwischen 0 und 50°C.

Als Hilfsstoffe seien beispielhaft genannt:
Zerfallsförderer wie Stärke, modifizierte Stärke, Zellulose, Zellulosederivate, quervernetztes Polyvinylpyrolidon (PVPP), Natriumalginat und kolloidales Siliciumdioxid; Bindemittel wie Gelatine, Tragant, Glukosesirup, Stärkekleister, Polyvinylpyrrolidone (PVP), Zellulosederivate, Polyethylenglykol MG 1000-5000 (PEG), und Alginate; Schmiermittel wie Magnesiumstearat, Calciumstearat, Stearinsäure, Paraffin, Talkum, pflanzliche oder tierische Fette, Öle und Wachse, Polyethylenglykol MG 1000-5000 und Silikone.

Als Füllstoffe seien beispielhaft genannt:
Calciumsulfat, Calciumcarbonat, di- und tribasische Calciumphosphate, Magnesiumcarbonat, Magnesiumhydroxycarbonat, Natriumchlorid, Natrium- und Kalium-zitrate, Tartrate und Succinate, Stärke, modifizierte Stärke, Zellulose, Zellulosepulver, Zucker wie Lactose, Saccharose oder Dextrose und Zuckeralkohole wie Mannit oder Sorbit.

Von besonderem Interesse sind Hilfs- und Füllstoffe aus der Gruppe Stärke, modifizierte Stärke wie Stärkekleister, Zellulose, PVP, PVPP, kolloidales Siliciumdioxid, Lactose, Magnesiumcarbonat, Magnesiumstearat und Calciumstearat.

Bei Kenntnis des Standes der Technik ist es ausgesprochen überraschend, daß durch die gemeinsame Granulation eines schwer löslichen Dihydropyridins mit einem der genannten ß-Blockern eine signifikante Erhöhung der Freisetzung und damit eine signifikante Verbesserung der Bioverfügbarkeit für das Dihydropyridin erreicht werden kann. Dieser erfindungsgemäße Effekt ist nicht abhängig von der Anwesenheit zusätzlicher Hilfsstoffe, die nach Kenntnis nach Standes der Technik die Freisetzungsrate erhöhen wie z.B. Komplexbildnern, oberflächenaktiven Stoffen oder wasserlöslichen Polymeren.

Als schwerlösliche Dihydropyridine seien Dihydropyridine genannt, deren Löslichkeit maximal 20 mg pro Liter Wasser bei 25°C beträgt.

Im Freisetzungstest nach der USP-Paddle-Methode unter den folgenden Bedingungen

4000 ml 0,1 N-Salzsäure

Paddle-Drehzahl 100 U/Min.

wurden nach 2 Stunden Freisetzungsraten für Nitrendipin von ca. 80 % erreicht für erfindungsgemäße Kombinationspräparate (Produkt gemäß Beispiel 1). Eine entsprechende Zubereitung einer herkömmlichen Nitrendipintablette, in welcher das Nitrendipin in gleicher kristalliner Zusammensetzung eingesetzt wurde und die die gleichen Hilfsstoffe enthält, zeigt nach 2 Stunden nur eine Freisetzungsrate von ca. 40 %. Der Verlauf der Freisetzung ist aus Tabelle 1 ersichtlich.

**Tabelle 1**

In vitro - Freisetzung von nitrendipinhaltigen Tabletten (USP-Paddle-Methode)

| Freisetzungszeit /h/ | kumulative Freisetzung von Nitrendipin in Prozent der eingesetzten Dosis | |
|---|---|---|
| | Tablette Beispiel 1 | Tablette Vergleichsbeispiel |
| 0,25 | 24 | 8 |
| 0,5 | 52 | 19 |
| 1 | 68 | 36 |
| 2 | 82 | 43 |

**Vergleichsbeispiel**

100 g Nitrendipin, 910 g Maisstärke, 680 g Cellulosepulver und 550 g Calciumcarbonat und 10 g Natriumlaurylsulfat werden in einem Planetenmischer 5 Minuten gemischt und mit einer wäßrigen Polyvinylpyrrolidon-Lösung (Feststoffgehalt 50 g) 10 Minuten granuliert. Die feuchte Masse wird durch ein Sieb gegeben und in einem Wirbelschichttrockner auf einen Endwassergehalt von ca. 4,5 % getrocknet. Das getrocknete Granulat wird durch Siebung (0,8 mm-Siebmaschenweite) egalisiert und intensiv mit 50 g Magnesiumstearat vermischt. Anschließend wird das Granulat zu 235 mg schweren Tabletten verpreßt.

Ausführungsbeispiele:

Beispiel 1

100 g Nitrendipin, 500 g Atenolol, 660 g Maisstärke. 430 g Cellulosepulver, 550 g Calciumcarbonat und 10 g Natriumlaurylsulfate werden in einem Planetenmischer 5 Minuten gemischt und mit einer wäßrigen Polvinylpyrrolidon-Lösung (Feststoffgehalt 50 g) 10 Minuten granuliert. Die feuchte Masse wird durch ein Sieb gegeben und in einem Wirbelschichttrockner auf einen Endwassergehalt von ca. 4,5 % getrocknet. Das getrocknete Granulat wird durch Siebung (0,8 mm-Siebmaschenweite) egalisiert und intensiv mit 50 g Magnesiumstearat vermischt. Anschließend wird das Granulat zu 235 mg schweren Tabletten verpreßt. Die Tabletten können mit einem Schutzlack überzogen werden.

Beispiel 2

50 g Nisoldipin, 2216 g Acebutololhydrochlorid, 2244 g Maisstärke, 250 g vorverkleisterte Maisstärke und 750 g Cellulosepulver werden in einem Mischgranulator mit einer wäßrigen Tween 80-Lösung (Gehalt 15 g) granuliert. Danach wird die feuchte Masse durch eine Raspel (Lochweite 4,0 mm) gegeben und in einem Umlufttrockenschrank auf ca. 3,8 % Endfeuchte getrocknet. Das getrocknete Granulat wird durch Siebung (1,25 mm-Siebmaschenweite)

egalisiert und intensiv mit 15 g Magnesiumstearat vermischt. Anschließend wird das Granulat zu 277 mg schweren Tabletten verpreßt.

Die Tabletten können mit einer Lichtschutzumhüllung überzogen werden. Alternativ kann das Granulat in Kapseln abgefüllt werden.

Patentansprüche

1. Festes Kombinationspräparat mit verbesserter Bioverfügbarkeit, enthaltend 1 Gewichtsteil eines schwerlöslichen Dihydropyridins der allgemeinen Formel (I)

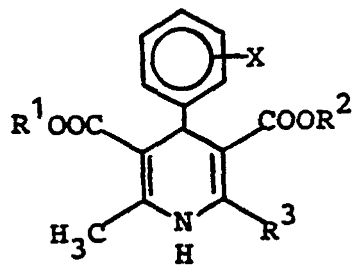

(I)

in der

$R^1$ $C_1$-$C_4$-Alkyl, gegebenenfalls substituiert durch $C_2$-$C_3$-Alkoxy,

$R^2$ $C_1$-$C_{10}$-Alkyl, gegebenenfalls substituiert durch $C_1$-$C_3$-Alkoxy, Trifluormethyl, N-Methyl-N-benzylamino,

$R^3$ $C_1$-$C_4$-Alkyl, Cyano, Hydroxymethyl und

X 2- bzw. 3-Nitro, 2,3-Dichlor, 2,3 Ringglied bestehend aus =N-O-N=,

bedeuten,

und 0,5 bis 10 Gewichtsteile eines ß-Blockers und gegebenenfalls übliche Hilfs- und Trägerstoffe.

2. Kombinationspräparat gemäß Anspruch 1, enthaltend einen ß-Blocker aus der Gruppe Acebutolol, Atenolol, Nadolol, Propranolol, Pindolol und Timolol.

3. Kombinationspräparat gemäß den Ansprüchen 1 und 2, enthaltend ein Dihydropyridin aus der Gruppe Nisoldipin, Nimodipin oder Nitrendipin.

4. Kombinationspräparat gemäß Ansprüchen 1 bis 3, enthaltend 1 Gewichtsteil des schwerlöslichen Dihydropyridins der Formel I, in welcher $R^1$ und $R^2$ immer voneinander verschieden sind und 1 bis 5 Gewichtsteile eines ß-Blockers.

5. Kombinationspräparat gemäß den Ansprüchen 1 bis 4, enthaltend Acebutolol oder Atenolol als ß-Blocker.

6. Kombinationspräparat gemäß den Ansprüchen 1 bis 5 in Form von Granulat.

7. Verfahren zur Herstellung von Kombinationspräparaten gemäß Anspruch 1, dadurch gekennzeichnet, daß man 1 Gewichtsteil des schwerlöslichen Dihydropyridins der allgemeinen Formel (I) und 0,5 bis 10 Gewichsteile des ß-Blockers gemeinsam mit Hilfs- und Füllstoffen granuliert und gegebenenfalls dieses Granulat zu üblichen Applikationsformen weiterverarbeitet.

8. Verfahren gemäß Anspruch 7, dadurch gekennzeichnet, daß man 1 Gewichtsteil Dihydropyridin und 1 bis 5 Gewichtsteile ß-Blocker mit Hilfs- und Füllstoffen

aus der Gruppe Stärke, Cellulose, PVP, Siliciumdioxid, Magnesiumcarbonat und/oder Lactose in einem Granulator wäßrig granuliert und das erhaltene Granulat gegebenenfalls in eine übliche Applikationsform überführt.

9. Verwendung von Kombinationspräparaten gemäß Anspruch 1 bei der Bekämpfung von Kreislauferkrankungen.

10. Verfahren zur Herstellung von Präparaten gemäß den Ansprüchen 1 bis 6, dadurch gekennzeichnet, daß man Ylidenverbindungen der allgemeinen Formel II, in welcher X und $R^1$ die in Anspruch 1 angegebene Bedeutung haben, mit Enaminverbindungen der allgemeinen Formel III, in welcher $R^2$ und $R^3$ die in Anspruch 1 angegebene Bedeutung haben, in niederen aliphatischen Alkoholen mit 1 bis 4 C-Atomen bei Temperaturen zwischen 40 und 100°C umsetzt; und anschließend das erhaltene Dihydropyridin gemäß dem Verfahren nach Anspruch 7 weiterverarbeitet.